# EUROPEAN PATENT APPLICATION

(11) **EP 3 795 691 A1**
(43) Date of publication of application: **24.03.2021**
(21) Application number: 19804372.1
(22) Date of filing: 19.04.2019
(51) Int. Cl.: C12P 7/64, A23D 9/007, A23D 9/04, C12R 1/645

(54) **METHOD FOR ADJUSTING COMPONENTS OF FATTY ACID COMPOSITION IN MICROBIAL OIL OF MORTIERELLA**

(30) Priority: 17.05.2018 CN 201810475952; 03.08.2018 CN 201810879600; 11.09.2018 CN 201811058560
(71) Applicant: Liang, Yun, Changsha, Hunan 410208 (CN)
(72) Inventor: QU, Hanpeng, Changsha, Hunan 410208 (CN); CAO, Sheng, Changsha, Hunan 410208 (CN); WANG, Shenjian, Changsha, Hunan 410208 (CN)
(74) Representative: Vitina, Maruta
(86) International application number: PCT/CN2019/083419
(87) International publication number: WO 2019/218841

(57) **Abstract**

Disclosed is a method for adjusting a fatty acid composition in a microbial oil from *mortierella.* During a fermentation, a carbon-to-nitrogen ratio of in a medium is reduced from (2.5-25):1 to 0. A consumption of carbon sources and a supply of carbon sources provide a growing condition for auxotroph, which contributes to a conversion of fatty acid. Meanwhile, during 0-120 hours of the fermentation, a culture temperature is controlled at 29-29°C; after 120 hours of the fermentation, the culture temperature is controlled at 15-28°C. Winterizations with temperature vibration and program temperature control are adopted during a refining process of the oil, which removes the fatty acid with high saturation, increase the relative content of the polyunsaturated fatty acid, and improves the low-temperature fluidity of the oil. A composition made by the method is suitable for preparing infant formula, nutraceuticals, health food or ordinary food.

## Description

### TECHNICAL FIELD

The present disclosure relates to microbial fermentations, and more particularly to a method for adjusting a fatty acid composition in a microbial oil from *mortierella.*

### BACKGROUND

Microbial oils, also named single cell oils are produced by oleaginous microorganisms such as yeasts, fungi and microalgae using carbon and nitrogen sources, and trace elements.

Oleaginous microorganisms are sources that are abundant and can grow under various culture conditions. It is promising in terms of industrial production of microbial oils. Microbial oil includes high levels of polyunsaturated fatty acids, for example, docosahexaenoic acid (DHA) and arachidonic acid (ARA), which are essential fatty acids for human to maintain important physiological functions.

A lack of ARA and DHA may cause permanent mental retardation and visual impairment in infants, and pruritus, dry eyes and distraction in class for children aged 6-12. The ARA and DHA are also effective in the prevention and treatment of hypertension, hyperlipidemia, diabetes and viral infection.

DHA and ARA products commercially available are mainly extracted from deep-sea fish oil, with unstable composition of polyunsaturated fatty acids. Due to the limitation on the source of raw materials, the manufacture of such products is costly and low-yield, and the realization of industrial production is impossible. Therefore, microbe oil has become an important resource to produce high-value fatty acids, such as linolenic acid (GLA), ARA, eicosapentaenoic acid (EPA) and DHA.

The microbial oils, produced by fermentation using *mortierella,* have a relatively high content of ARA. However, in the prior art, the microbial oils, produced by fermentation using *mortierella,* have a relatively low content of ARA and have some harmful fatty acids such as myristic acid, lauric acid and erucic acid, which hinders the use of microbial oils.

### SUMMARY

The object of the present invention is to provide a method for adjusting a fatty acid composition in a microbial oil from *mortierella* to solve the problem that *mortierella* microbe oils have a high content of harmful fatty acids and saturated fatty acids and a low content of ARA. The method adjusts a fatty acid composition in a microbial oil from *mortierella* so as to produce a microbial oil having a high ARA content and a low content of harmful fatty acids. The microbial oil is suitable for producing infant food and nutraceuticals.

In order to realize the above-mentioned object, in a first aspect, the present invention provides a method for adjusting a fatty acid composition in a microbial oil from *mortierella,* comprising:
during a fermentation, reducing a carbon-to-nitrogen ratio in a medium from (2.5-25:1) to 0;
during 0-120 hours of the fermentation, controlling a culture temperature at 29-29°C; and
after 120 hours of the fermentation, controlling the culture temperature at 15-28°C.

At an early stage of the fermentations, a high temperature and a high carbon-to-nitrogen ratio enable microbial cells to rapidly grow and reproduce, largely increasing a biomass and accelerating a conversion and enrichment of the microbial oil. At a later stage of the fermentation, a low temperature and a low carbon-to-nitrogen ratio promote a further conversion of fatty acids and restrict a content of non-essential fatty acids and harmful fatty acids.

In some embodiments, the controlling the carbon-to-nitrogen ratio in the medium comprises:
during 0-40 hours of the fermentation, controlling the carbon-to-nitrogen ratio at (2.5-25): 1;
during 41-65 hours of the fermentation, controlling the carbon-to-nitrogen ratio at (2-10): 1;
during 66-89 hours of the fermentation, controlling the carbon-to-nitrogen ratio at (2-8):1;
during 90-113 hours of the fermentation, controlling the carbon-to-nitrogen ratio d at (1-5): 1;
during 114-122 hours of the fermentation, controlling the carbon-to-nitrogen ratio at (1-2): 1;
during 123-130 hours of the fermentation, controlling the carbon-to-nitrogen ratio at (1-0.5):1; and
after 130 hours of the fermentation, controlling the carbon-to-nitrogen ratio to 0.

In some embodiments, the controlling the carbon-to-nitrogen ratio in the medium is performed by regularly supplying a carbon resource as needed to the medium, while a nitrogen resource is only added into the medium at beginning.

In some embodiments, the method further comprises a second winterization in a refining process of the oil, and the second winterization comprises:
heating the oil to 70-90°C to dewater the microbial oil;
cooling the oil to 20-30°C;
performing an ambient-temperature winterization on the oil for 16-24 hours;
filtering the oil and dewatering the oil; and
cooling the oil according to a set program to -10-1°C to perform a low-temperature winterization on the oil for 48-90 hours.

In a second aspect, the present invention provides a microbial oil from *mortierella* produced by any one of the above-mentioned the methods. the microbial oil from *mortierella* comprises: based on a total weight of the microbial oil,
no less than 40 wt% of an arachidonic acid;
0-5 wt% of a C12:0 fatty acid;
0-5 wt% of C14:0 and C14:1 fatty acids;
1-25 wt% of C16:0 and C16:1 fatty acids;
2-35 wt% of C18:0, C18:1 and C18:2 fatty acids; and
0-5 wt% of a C22:1, n-9 fatty acid.

In some embodiments, a weight ratio of unsaturated fatty acids to saturated fatty acids in the microbial oil from *mortierella* is not less than 0.6. Preferably, the weight ratio of the unsaturated fatty acids to the saturated fatty acids in the microbial oil from *mortierella* is not less than 2.3.

In a third aspect, the present invention provides a composition comprising any one of the above-mentioned microbial oil from *mortierellas,* and the composition is suitable for infant formula, nutraceuticals, health food and ordinary food.

The above-mentioned technical solutions improve a yield of a microbial oil, and the microbial oil has a high content of ARA and a low content of harmful fatty acids such as myristic acid, lauric acid and erucic acid. The microbial oil is clear and transparent at -10-5°C.

### DETAILED DESCRIPTION OF EMBODIMENTS

It should be noted that endpoints and values within ranges disclosed herein are only exemplary, and are intended to include any values close to these values. Any possible combination of numerical values within the range to form one or more new ranges should be considered to be expressly disclosed in this disclosure.

In examples and comparative examples of the present invention, *mortierella* microorganism is *mortierella alpina.* All strains and various culture supplies used are commercially available, and all reagents and detection methods involved are implemented in accordance with national standards.

### Example 1

1) Production strains: Original strains were inoculated and cultured in a shake flask containing a sterilized and cooled culture medium.
A formula of the culture medium was: 3 wt% of glucose and 1.5 wt% of yeast powder. The pH of the culture medium was controlled at 8.0-8.5 with sodium hydroxide aqueous solution.
Flasks were incubated on a shaker at 28±1°C for 36-44 hours at a speed of 200 rpm. The strains were then transferred to a primary seed tank upon the formation of mycelium.
2) Primary inoculum: The strains were inoculated and cultured in the primary seed tank containing a sterilized and cooled culture medium to obtain a primary inoculum.
A formula of the culture medium was: 3 wt% of glucose, 1.5 wt% of yeast powder, 0.06 wt% of defoamer (epoxy silicone ether), and 0.09 wt% of sodium hydroxide.
The primary seed tank was incubated at 29±1°C for 48 hours at a speed of 200 rpm. A ventilation was at 0.55 vvm.
3) Secondary inoculum: The strains were inoculated and cultured in a secondary seed tank containing a sterilized and cooled culture medium to obtain a secondary inoculum.
A formula of the culture medium was: 4 wt% of glucose, 1.5 wt% of yeast powder, 0.095 wt% of defoamer (epoxy silicone ether), and 0.11 wt% of sodium hydroxide.
The secondary seed tank was incubated at 29±1°C for 48 hours at a speed of 200 rpm. A ventilation was at 0.55 vvm.
4) Fermentation: 15% of the secondary inoculum was inoculated into a fermentor containing a fermentation medium.
A formula of the culture medium was: 8 wt% of glucose, 2 wt% of yeast powder, and 0.04 wt% of defoamer (epoxy silicone ether).
The fermentor was incubated at 30°C for hours at a speed of 160 rpm. A ventilation was at 0.8 vvm. After 80 hours, the fermentation was incubated at 22°C. The ventilation was at 0.67 vvm. By addition of a sterile glucose solution containing 25% glucose, a carbon-to-nitrogen ratio of was controlled. Table 1 showed during 7 days of fermentation, the adjustments of carbon-to-nitrogen ratio and pH value of the fermentation medium.

**Table 1**

| Time(h) | 0-40 | 41-65 | 66-89 | 90-113 | 114-122 | 123-130 | ≥131 |
|---|---|---|---|---|---|---|---|
| C/N | 10-15 | 6-8 | 4-6 | 2-4 | 1-2 | 0.5-1 | 0 |
| pH | without adjustment | 6.2-6.5 | 6.5-6.7 | 6.7-7.0 | 7.0-7.4 | 7.0-7.4 | 7.4-8.0 |

5) Filtration: The mycelium was separated from water by a plate and frame press, and the separated mycelium was crushed and granulated by an oscillating granulator.
6) Drying: The mycelium was dried by a fluidized drying tower under hot air of 180°C, and a moisture content of the mycelium after drying was ≤5%.
7) Extraction: The dried mycelium was extracted with butane to obtain a crude oil.
8) Water washing and degumming: The crude oil was added with pure water which was 10% by weight of the crude oil. Then the oil was heated to 85°C and stirred at 80 rpm for 20 min. The oil was stood for 2 hours and was separated from water.
9) Acid refining: The oil was heated to 75°C, added with citric acid which was 4‰ by weight of the oil, and stirred at 80 rpm for 40 min. Then the oil was added with hot water at 85°C which was 10% by weight of the oil, and stirred for 20 min. The oil was stood for 3 hours and was separated from water.
10) Alkali refining: The oil was heated to 45°C and added with alkali solution according to an acid value of the oil. (The addition=7.13×10⁻⁴× the acid value×the oil weight). The oil was added with 40% sodium hydroxide aqueous solution and stirred at 80 rpm for 50 min. Then the oil was heated to 80°C and added with pure water at 85°C which was 5% by weight of the oil, stirred at 80 rpm for 15 min. The oil was then centrifuged by a two-phase centrifuge to remove saponins.
11) Dehydration: The oil was heated to 85°C and dehydrated at -0.1 MPa for 35 min.
12) Ambient-temperature winterization: The dehydrated oil was naturally cooled to 25°C for nucleation. The ambient-temperature winterization lasted for 20 hours.
13) Filtration: The winterized oil was filtered by a plate and frame filter press at a pressure of 0.3 MPa. A filter medium was industrial filter cloth.
14) Second dehydration: The filtered oil was heated to 85°C and dehydrated at -0.1 MPa for 35 min.
15) Low-temperature winterization: The dehydrated oil was cooled according to a set program. The dehydrated oil was cooled at a rate of 20°C/h to 45°C, and then cooled at a rate of 3°C/h. The cooling rate was gradually decreased to 1°C/h. When the oil temperature was 15°C, the oil was reheated at a rate of 1.5°C/h for 5 hours for crystal growth. After that, the oil was cooled at a rate of 2°C/h to -5°C. The crystal growth lasted for at least 16 hours. The low-temperature winterization lasted for 80 hours.
16) Second filtration: The winterized oil was filtered by a plate and frame filter press at a pressure of 0.2 MPa. A filter medium was industrial filter cloth.
17) Decolorization: The oil was decolorized by activated carbon and activated clay for 70 min. The activated carbon was 1.5% by weight of the oil, and the activated clay was 1.5% by weight of the oil.
18) Deodorization: The decolorized oil was deodorized at 175±2°C for 4 hours. A steam pressure was maintained at 0.2-0.3 MPa. A vacuum degree is at 50Pa, and a steam consumption was controlled at about 5% by weight of the oil.

### Example 2

1) Production strains: Original strains were inoculated and cultured in a shake flask containing a sterilized and cooled culture medium.
A formula of the culture medium was: 3 wt% of glucose and 1.5 wt% of yeast powder. The pH of the culture medium was controlled at 8.0-8.5 with sodium hydroxide aqueous solution.
Flasks were incubated on a shaker at 28±1°C for 44-50 hours at a speed of 200 rpm. The strains were then transferred to a primary seed tank upon the formation of mycelium.
2) Primary inoculum: The strains were inoculated and cultured in the primary seed tank containing a sterilized and cooled culture medium to obtain a primary inoculum.
A formula of the culture medium was: 3 wt% of glucose, 1.5 wt% of yeast powder, 0.06 wt% of defoamer (epoxy silicone ether), and 0.09 wt% of sodium hydroxide.
The primary seed tank was incubated at 29±1°C for 48 hours at a speed of 200 rpm. A ventilation was at 0.55 vvm.
3) Secondary inoculum: The strains were inoculated and cultured in a secondary seed tank containing a sterilized and cooled culture medium to obtain a secondary inoculum.
A formula of the culture medium was: 4 wt% of glucose, 1.5 wt% of yeast powder, 0.095 wt% of defoamer (epoxy silicone ether), and 0.11 wt% of sodium hydroxide.
The secondary seed tank was incubated at 29±1°C for 48 hours at a speed of 200 rpm. A ventilation was at 0.55 vvm.
4) Fermentation: 10% (v/v) of the secondary inoculum was inoculated into a fermentor containing a fermentation medium.
A formula of the culture medium was: 8 wt% of glucose, 2 wt% of yeast powder, and 0.03 wt% of defoamer (epoxy silicone ether).
The fermentor was incubated at 29°C for hours at a speed of 120 rpm. A ventilation was at 0.75 vvm. After 80 hours, the fermentation was incubated at 25°C. The ventilation was at 0.65 vvm. By addition of a sterile glucose solution containing 25% glucose, a carbon-to-nitrogen ratio of was controlled. Table 2 showed during 7 days of fermentation, the adjustments of carbon-to-nitrogen ratio and pH value of the fermentation medium.

**Table 2**

| Time(h) | 0-40 | 41-65 | 66-89 | 90-113 | 114-122 | 130-144 | ≥144 |
|---|---|---|---|---|---|---|---|
| C/N | 2.5-5 | 2-2.5 | 2-2.5 | 1-2 | 1-1.5 | 0.5-1 | 0 |
| pH | Without adjustment | 6.2-6.5 | 6.5-6.7 | 6.7-7.0 | 7.0-7.4 | 7.0-7.4 | 7.4-8.0 |

5) Filtration: The mycelium was separated from water by a plate and frame press, and the separated mycelium was crushed and granulated by an oscillating granulator.
6) Drying: The mycelium was dried by a fluidized drying tower under hot air of 180°C, and a moisture content of the mycelium after drying was ≤5%.
7) Extraction: The dried mycelium was extracted with butane to obtain a crude oil.
8) Water washing and degumming: The crude oil was added with pure water which was 10% by weight of the crude oil. Then the oil was heated to 85°C and stirred at 80 rpm for 20 min. The oil was stood for 2 hours and was separated from water.
9) Acid refining: The oil was heated to 75°C, added with citric acid which was 4‰ by weight of the oil, and stirred at 80 rpm for 40 min. Then the oil was added with hot water at 85°C which was 10% by weight of the oil, and stirred for 20 min. The oil was stood for 3 hours and was separated from water.
10) Alkali refining: The oil was heated to 75°C and added with alkali solution according to an acid value of the oil. (The addition=7.13×10⁻⁴×acid value× oil weight). The oil was added with 40% sodium hydroxide aqueous solution and stirred at 80 rpm for 50 min. Then the oil was heated to 80°C and added with pure water at 85°C which was 5% by weight of the oil, stirred at 80 rpm for 15 min. The oil was then centrifuged by a two-phase centrifuge to remove saponins.
11) Dehydration: The oil was heated to 70°C and dehydrated at -0.1 MPa for 35 min.
12) Ambient-temperature winterization: The dehydrated oil was naturally cooled to 20°C for nucleation. The ambient-temperature winterization lasted for 16 hours.
13) Filtration: The winterized oil was filtered by a plate and frame filter press at a pressure of 0.3 MPa. A filter medium was industrial filter cloth.
14) Second dehydration: The filtered oil was heated to 85°C and dehydrated at -0.1 MPa for 35 min.
15) Low-temperature winterization: The dehydrated oil was cooled according to a set program. The dehydrated oil was cooled at a rate of 20°C/h to 45°C, and then cooled at a rate of 3°C/h. The cooling rate was gradually decreased to 1°C/h. When the oil temperature was 13°C, the oil was reheated at a rate of 1°C/h for 4 hours for crystal growth. After that, the oil was cooled at a rate of 1°C/h to 1°C. The crystal growth lasted for at least 16 hours. The low-temperature winterization lasted for 48 hours.
16) Second filtration: The winterized oil was filtered by a plate and frame filter press at a pressure of 0.2 MPa. A filter medium was industrial filter cloth.
17) Decolorization: The oil was decolorized by activated carbon and activated clay for 70 min. The activated carbon was 1.5% by weight of the oil, and the activated clay was 1.5% by weight of the oil.
18) Deodorization: The decolorized oil was deodorized at 175±2°C for 4 hours. A steam pressure was maintained at 0.2-0.3 MPa. A vacuum degree is at 50Pa, and a steam consumption was controlled at about 5% by weight of the oil.

### Example 3

1) Production strains: Original strains were inoculated and cultured in a shake flask containing a sterilized and cooled culture medium.
A formula of the culture medium was: 3 wt% of glucose and 1.5 wt% of yeast powder. The pH of the culture medium was controlled at 8.0-8.5 with sodium hydroxide aqueous solution.
Flasks were incubated on a shaker at 28±1°C for 44-50 hours at a speed of 200 rpm. The strains were then transferred to a primary seed tank upon the formation of mycelium.
2) Primary inoculum: The strains were inoculated and cultured in the primary seed tank containing a sterilized and cooled culture medium to obtain a primary inoculum.
A formula of the culture medium was: 3 wt% of glucose, 1.5 wt% of yeast powder, 0.06 wt% of defoamer (epoxy silicone ether), and 0.09 wt% of sodium hydroxide.
The primary seed tank was incubated at 29±1°C for 48 hours at a speed of 200 rpm. A ventilation was at 0.55 vvm.
3) Secondary inoculum: The strains were inoculated and cultured in a secondary seed tank containing a sterilized and cooled culture medium to obtain a secondary inoculum.
A formula of the culture medium was: 4 wt% of glucose, 1.5 wt% of yeast powder, 0.095 wt% of defoamer (epoxy silicone ether), and 0.11 wt% of sodium hydroxide.
The secondary seed tank was incubated at 29±1°C for 48 hours at a speed of 200 rpm. A ventilation was at 0.55 vvm.
4) Fermentation: 20% (v/v) of the secondary inoculum was inoculated into a fermentor containing a fermentation medium.
A formula of the culture medium was: 8 wt% of glucose, 2 wt% of yeast powder, and 0.04 wt% of defoamer (epoxy silicone ether).
The fermentor was incubated at 32°C for hours at a speed of 200 rpm. A ventilation was at 0.9 vvm. After 80 hours, the fermentation was incubated at 28°C. The ventilation was at 0.7 vvm. By addition of a sterile glucose solution containing 25% glucose, a carbon-to-nitrogen ratio of was controlled. Table 3 showed during 7 days of fermentation, the adjustments of carbon-to-nitrogen ratio and pH value of the fermentation medium.

**Table 3**

| Time(h) | 0-40 | 41-65 | 66-89 | 90-113 | 114-122 | 123-130 | ≥131 |
|---|---|---|---|---|---|---|---|
| C/N | 20-25 | 8-10 | 6-8 | 4-5 | 1.5-2 | 0.5-1 | 0 |
| pH | Without adjustment | 6.2-6.5 | 6.5-6.7 | 6.7-7.0 | 7.0-7.4 | 7.0-7.4 | 7.4-8.0 |

5) Filtration: The mycelium was separated from water by a plate and frame press, and the separated mycelium was crushed and granulated by an oscillating granulator.
6) Drying: The mycelium was dried by a fluidized drying tower under hot air of 185°C, and a moisture content of the mycelium after drying was ≤5%.
7) Extraction: The dried mycelium was extracted with butane to obtain a crude oil.
8) Water washing and degumming: The crude oil was added with pure water which was 10% by weight of the crude oil. Then the oil was heated to 85°C and stirred at 80 rpm for 20 min. The oil was stood for 2 hours and was separated from water.
9) Acid refining: The oil was heated to 75°C, added with citric acid which was 4‰ by weight of the oil, and stirred at 80 rpm for 40 min. Then the oil was added with hot water at 85°C which was 10% by weight of the oil, and stirred for 20 min. The oil was stood for 3 hours and was separated from water.
10) Alkali refining: The oil was heated to 45°C and added with alkali solution according to an acid value of the oil. (The addition=7.13×10⁻⁴×acid value× oil weight). The oil was added with 40% sodium hydroxide aqueous solution and stirred at 80 rpm for 50 min. Then the oil was heated to 80°C and added with pure water at 85°C which was 5% by weight of the oil, stirred at 80 rpm for 15 min. The oil was then centrifuged by a two-phase centrifuge to remove saponins.
11) Dehydration: The oil was heated to 90°C and dehydrated at -0.1 MPa for 35 min.
12) Ambient-temperature winterization: The dehydrated oil was naturally cooled to 30°C for nucleation. The ambient-temperature winterization lasted for 24 hours.
13) Filtration: The winterized oil was filtered by a plate and frame filter press at a pressure of 0.3 MPa. A filter medium was industrial filter cloth.
14) Second dehydration: The filtered oil was heated to 90°C and dehydrated at -0.1 MPa for 35 min.
15) Low-temperature winterization: The dehydrated oil was cooled according to a set program. The dehydrated oil was cooled at a rate of 20°C/h to 45°C, and then cooled at a rate of 3°C/h. The cooling rate was gradually decreased to 1°C/h. When the oil temperature was 12°C, the oil was reheated at a rate of 2°C/h for 5 hours for crystal growth. After that, the oil was cooled at a rate of 2°C/h to -10°C. The crystal growth lasted for at least 16 hours. The low-temperature winterization lasted for 90 hours.
16) Second filtration: The winterized oil was filtered by a plate and frame filter press at a pressure of 0.2 MPa. A filter medium was industrial filter cloth.
17) Decolorization: The oil was decolorized by activated carbon and activated clay for 70 min. The activated carbon was 1.5% by weight of the oil, and the activated clay was 1.5% by weight of the oil.
18) Deodorization: The decolorized oil was deodorized at 175±2°C for 4 hours. A steam pressure was maintained at 0.2-0.3 MPa. A vacuum degree is at 50Pa, and a steam consumption was controlled at about 5% by weight of the oil. The oil was cooled and the vacuum was broken to obtain a product oil.

### Comparative Example

A method used herein was basically the same as that used in Example 1 except that a formula of a fermentation medium was: 5 wt% of glucose, 2.5 wt% of sodium glutamate, and 1.0 wt% of yeast extract (a carbon-to-nitrogen ratio was 11.8:1); strains was thermostatically cultured at 28°C for 7 days; and the oil was rapidly cooled to -5°C for 48 hours for crystal growth.

### Results and Comparison

Oil products of Examples 1-3 and Comparative example were detected by gas chromatography for analysis of fatty acid composition. Weight percentage of individual fatty acids in respective oil products were shown in Table 4.

**Table 4**

| Name | Example 1 | Example 2 | Example 3 | Comparative Example |
|---|---|---|---|---|
| Lauric acid (C12:0) | 0.02 | 0.028 | 0.03 | 1.12 |
| Myristic acid (C14:0) | 0.386 | 0.412 | 0.397 | 1.35 |
| Myristic acid (C14:1) | 0.025 | 0.031 | 0.028 | 1.28 |
| Palmitic acid (C16:0) | 6.25 | 6.35 | 6.34 | 27.47 |
| Palmitoleic acid C16:1 | 0.144 | 0.213 | 0.196 | 0.113 |
| Stearic acid (C18:0) | 5.776 | 5.634 | 4.236 | 17.36 |
| Oleic acid (C18:1, n-9) | 7.192 | 6.21 | 5.78 | 0.19 |
| Linoleic acid | 7.45 | 6.599 | 6.707 | 0.16 |
| (C18:2, n-6) | | | | |
| γ-linolenic acid (C18:3, n-6) | 3.949 | 3.672 | 3.11 | 0.13 |
| ARA (C20:4, n-6) | 51.7 | 54.76 | 56.9 | 35.87 |
| Arachidic acid (C22:0) | 3.411 | 3.42 | 3.395 | 4.12 |
| EPA (C20:5, n-3) | 0.099 | 0.087 | 0.095 | 0.12 |
| DPA (C22:5, n-6) | 0.02 | 0.03 | 0.023 | 0.20 |
| DHA (C22:6, n-3) | 0.022 | 0.031 | 0.025 | 0.032 |
| Tetracosanoic acid (C24:0) | 9.342 | 8.16 | 8.45 | 15.12 |
| Erucic acid (C22:1, n-9) | 0.114 | 0.153 | 0.123 | 0.353 |
| Other fatty acids | 4.1 | 4.21 | 4.165 | 4.71 |
| Unsaturated fatty acids: Saturated fatty acids | 2.41 | 2.54 | 2.66 | 0.59 |

Table 4 showed that the oil produced by the present method had a higher content of ARA and a lower content of harmful fatty acids. In addition, the oil produced by the method according to the present disclosure had better low temperature solidification performance.

The resulting microbial oil from *mortierella* has a high content of ARA. The preferred method of the present disclosure can produce a microbial oil from *mortierella* with over 50 wt% of ARA and a lower content of harmful fatty acids.

The resulting microbial oil has a high content of ARA and a low content of harmful fatty acids, and a good low temperature solidification performance. The microbial oil can be used to produce infant formula, especially milk powder. In addition, the microbial oil can also be applied in nutraceuticals for those who need ARA for health care, and health food and ordinary food as a supplement of daily intake.

The above-mentioned embodiments are only preferred embodiments, and not intend to limit the scope of the present invention. It should be noted that variations and modifications can be made without departing from the spirit of the invention should fall in the scope of the present invention.

## Claims

1. A method for adjusting a fatty acid composition in a microbial oil from *mortierella,* **characterized by** comprising:
during a fermentation, reducing a carbon-to-nitrogen ratio in a medium from (2.5-25:1) to 0;
during 0-120 hours of the fermentation, controlling a culture temperature at 29-29°C;
and
after 120 hours of the fermentation, controlling the culture temperature at 15-28°C.

2. The method according to claim 1, **characterized in that** the controlling the carbon-to-nitrogen ratio in the medium comprises:
during 0-40 hours of the fermentation, controlling the carbon-to-nitrogen ratio at (2.5-25): 1;
during 41-65 hours of the fermentation, controlling the carbon-to-nitrogen ratio at (2-10): 1;
during 66-89 hours of the fermentation, controlling the carbon-to-nitrogen ratio at (2-8):1;
during 90-113 hours of the fermentation, controlling the carbon-to-nitrogen ratio d at (1-5): 1;
during 114-122 hours of the fermentation, controlling the carbon-to-nitrogen ratio at (1-2): 1;
during 123-130 hours of the fermentation, controlling the carbon-to-nitrogen ratio at (1-0.5): 1; and
after 130 hours of the fermentation, controlling the carbon-to-nitrogen ratio to 0.

3. The method according to claim 1 or 2, **characterized in that** the controlling the carbon-to-nitrogen ratio in the medium is performed by regularly supplying a carbon resource as needed into the medium, while a nitrogen resource is only added into the medium at beginning.

4. The method according to claim 1, **characterized in that** the method further comprises a second winterization in a refining process of the oil, and the second winterization comprises:
heating the oil to 70-90°C to dewater the microbial oil;
cooling down the oil to 20-30°C;
performing an ambient-temperature winterization on the oil for 16-24 hours;
filtering the oil and dewatering the oil; and
cooling the oil according to a set program to -10-1°C to perform a low-temperature winterization on the oil for 48-90 hours.

5. A microbial oil from *mortierella,* **characterized in that** the microbial oil is produced through the method according to any one of claims 1-4, **characterized in that** the microbial oil comprises: based on a total weight of the microbial oil,
no less than 40 wt% of an arachidonic acid;
0-5 wt% of a C12:0 fatty acid;
0-5 wt% of C14:0 and C14:1 fatty acids;
1-25 wt% of C16:0 and C16:1 fatty acids;
2-35 wt% of C18:0, C18:1 and C18:2 fatty acids; and
0-5 wt% of a C22:1, n-9 fatty acid.

6. The microbial oil according to claim 5, **characterized in that** a weight ratio of unsaturated fatty acids to saturated fatty acids in the microbial oil is not less than 0.60.

7. The microbial oil according to claim 6, **characterized in that** the weight ratio of the unsaturated fatty acids to the saturated fatty acids in the microbial oil is not less than 2.3.

8. A composition comprising the microbial oil according to any one of claims 5-7, wherein the composition is suitable for preparing infant formula, nutraceuticals, health food or ordinary food.
